# EUROPÄISCHE PATENTSCHRIFT

(11) **EP 1 114 615 B1**
(45) Veröffentlichungstag und Bekanntmachung des Hinweises auf die Patenterteilung: **19.04.2006**
(21) Anmeldenummer: 01200032.9
(22) Anmeldetag: 04.01.2001
(51) Int. Cl.: A61B 6/00

(54) **Vorrichtung zur Darstellung des zeitlichen Verlaufs des Blutflusses**
Device for representing the temporal course of blood flow
Dispositif pour la représentation de l'évolution temporelle du débit sanguin

(30) Priorität: 05.01.2000 DE 10000185
(43) Veröffentlichungstag der Anmeldung: 11.07.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Koppe, Reiner Dr.,, Habsburgerallee 11 D-52064 Aachen (DE); Klotz, Erhard, Habsburgerallee 11 D-52064 Aachen (DE); Kuhn, Michael Dr.,, Habsburgerallee 11 D-52064 Aachen (DE); Op de Beek, John Dr.,, Habsburgerallee 11 D-52064 Aachen (DE)
(74) Vertreter: Volmer, Georg

(56) Entgegenhaltungen:
- EP-A- 0 587 334
- EP-A- 0 860 696
- EP-A- 0 880 109
- DE-C- 3 018 129

## Beschreibung

Die Erfindung betrifft eine Röntgeneinrichtung zur Darstellung des zeitlichen Verlaufs des Blutflusses in einem Untersuchungsobjekt nach dem Anspruch 1.

In DE 3018129 C1 wird eine Erstellung von Röntgen-Subtraktionsbildern beschrieben.

Aus der EP 860 696 A2 ist ein Verfahren bekannt, das es ermöglicht, verteilte Strukturen. z.B. ein mit Kontrastmittel gefülltes Gefäßsystem, in einem synthetischen Projektionsbild darzustellen, das die in einem vorgebbaren Teilvolumen befindlichen Teile des Gefäßsystems deutlicher wiedergibt als die aus unterschiedlichen Perspektiven aufgenommenen Röntgenaufnahmen, aus denen dieses Projektivasbild abgeleitet wird. Mit diesem Verfahren ergibt sich somit ein zeitlich gemitteltes "eingefrorenes" Bild des Gefaßbaumes, wobei der zeitliche Verlauf des Blutflusses nicht sichtbar ist.

Bei der dreidimensionalen Rotationsangiographie wird eine Serie von Röntgenprojektionsbildern des Untersuchungsobjekts aus unterschiedlichen Frojektionstichtungen erstellt, während ein Kontrastmittel in die Blutgefäße des Untersuchungsobjekts gespritzt wird. Aus diesen Röntgenprojektionsbildern wird mittels eines bekannten Rekonstruktionsalgorithmus, z.B. des Feldkamp-Algorithmus, ein dreidimensionales Bild erstellt, das zeitlich gemittelt den Gefaßbaum im Raum zeigt. Auch bei der zweidimensionalen Angiographie ist es bekannt, ein zweidimensionales "eingefrorenes" Bild des Gefäßbaumes zu erstellen.

Für verschiedene Anwendungen, wie die Analyse von Pathologien der zerebralen Gefäße, z.B. bei Gefil3mißbildungen (Stenosen, artiellen-venösen Mißbildungen), ist es jedoch von Bedeutung, den zeitlichen Verlauf des Blutflusses zu kennen und darstellen zu können. Der Erfindung liegt deshalb die Aufgabe zugrunde, eine Röntgeneinrichtung bereitzustellen, mit dem sich der zeitliche Verlauf des Blutflusses in einem Untersuchungsobjekt darstellen läßt. Dabei soll auch berücksichtigt werden, daß ein Kontrastmittel nicht mehrfach demselben Patienten in kurzer Zeit injiziert werden kann, so daß die Röntgeneinrichtung möglichst mit einer einzigen Kontrastmittelinjektion auskommen soll. Außerdem sollen die Bilder in möglichst kurzer Zeit, mit einem möglichst geringen Aufwand und in möglichst hoher Auflösung erstellbar sein.

Diese Aufgabe wird erfindungsgemäß durch die in Anspruch 1 angegebene Röntgeneinrichtung gelöst.

Die Erfindung geht dabei von der Erkenntnis aus, daß ein Bilddatensatz, der ein zweidimensionaler oder dreidimensionaler Bilddatensatz sein kann und Informationen über den Verlauf der Blutgefäße in dem Untersuchungsobjekt enthält, zeitlich kodiert werden kann, so daß er auch Informationen über den zeitlichen Verlauf des Blutflusses enthält Diese zeitliche Kodierung erfolgt erfindungsgemäß dadurch, daß der Bilddatensatz mit einer Serie von Röntgenprojektionsbildern verglichen wird, wobei die Röntgenprojektionsbilder zeitlich nacheinander erstellt wurden und jeweils die Information über die Verteilung eines injizierten Kontrastmittels in den Blutgefäßen zu einem anderen Zeitpunkt enthalten. Dadurch, daß jedes Röntgenprojektionsbild mit dem Bilddatensatz: einzeln verglichen wird, d.h- daß jeder Bildwert des Bilddatensatzes mit den Bildwetten der einzelnen Röntgenprojektionsbilder verglichen wird, wird quasi überprüft, welche Teile des in dem Bilddatensatz enthaltenen Gefäßbaumes jeweils zu den einzelnen Zeitpunkten, denen die Röntgenprojektionsbilder zugeordnet sind, mit Kontrastmittel gefüllt sind. Durch geeignete Darstellungsmethoden läßt sich der auf diese Weise zeitlich kodierte Bilddatensatz in ein oder mehrere Bilder umrechnen, die den zeitlichen Verlauf des Blutflusses darstellen. Aus jeweils zwei zeitlich nacheinander ermittelten Röntgenprojektionsbildern Eⱼ₋₁, Eⱼ werden in Verfahrensschritt 304 Differenzbilder Fⱼ mit dem bereits oben beschriebenen Informationsgehalt ermittelt. Diese Differenzbilder Fⱼ werden nun einzeln nacheinander mit dem segmentierten Bilddatensatz verglichen, d.h. es werden die Pixel Pₗ jedes einzelnen Differenzbildes Fⱼ mit den Pixeln Pₖ des segmentierten zweidimensionalen Bilddatensatzes H' verglichen. Dazu ist erforderlich, daß die Röntgenprojektionsbilder Eⱼ und der zweidimensionale Bilddatensatz mit derselben bildgebenden Einheit aus derselben Röntgenposition erfäßt wurde. Durch diesen Vergleich im Vedhbrennchntt 305 werden für jedes Differenzbild Fⱼ in Verfahrensschritt 306 ein zugehöriger Teilpixelsatz Mⱼ erstellt, der mit den weiter oben beschriebenen Teilvoxelsätzen Lⱼ vergleichbar ist und die Information enthält, welche Pixel des segmentierten Bilddateasxtzes H' einen Teil des Geäßsystemes zeigen, das sich in einem bestimmten Zeitintervall mit Kontrastmittel gefüllt hat bzw. aus dem in diesem Zeitintervall das Kontrastmittel abgenossen ist

Die Erfindung wird nachfolgend anhand der Zeichnungen näher erläutert. Es zeigen:
- Fig. 1: eine Röntgeneinrichtung zur Durchführung des erfindungemäßen Verfahrens,
- Fig. 2: ein Ablaufschema einer ersten Ausgestaltung des erfindungsgemäßen Verfahrens,
- Fig. 3: eine Skizze zur Erläuterung der zeitlichen Kodierung, und
- Fig. 5: ein Ablaufschema einer dritten Ausgestaltung des erfindungsgemäßen Verfahrens.

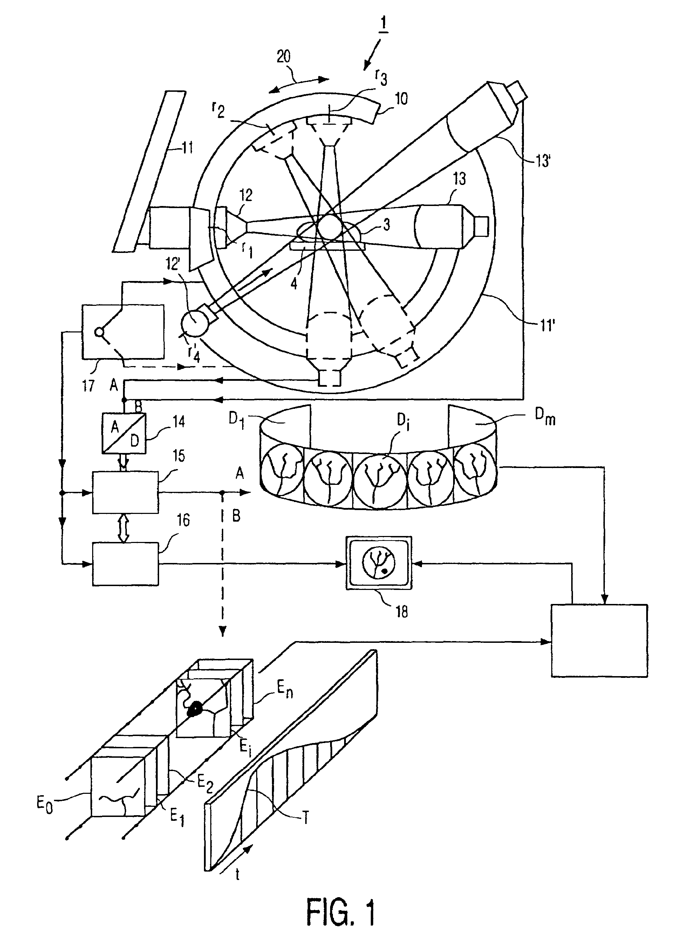

In Fig. 1 ist eine Röntgmeinfichmng 1 gezeigt, die der Erstellung zweidimensionaler Röntgenaufnahmen eines auf einem Tisch 4 befindlichen Untersuchungsobjekts 3, beispielsweise eines Patienten, dient. Diese weist eine Röntgenquelle 12 und einen Röntgendetektor 13 auf, die aufeinander ausgerichtet und an einem C-Bogen 10 befestigt sind, der seinerseits an einem nur tdiweise datgestellten Stativ 11 gehaltert ist Der C-Bogen 10 kann einerseits um eine waagerechte Achse geschwenkt und andererseits mittels eines nicht näher dargescellten Motorantnebs in Richtung des Doppelpfeils 20 um z.B. 180° um seinen Mittelpunkt bewegt werden. Bei dieser Beweguag kann eine Vielzahl von Röntgenaumahmen erzeugt werden, die das Untersuchungsobjekt 3 aus verschiedenen reproduzierbaren Perspektiven bzw. Röntgenpositianen r₁, r₂, r₃ der bildgebenden Einheit 12, 13 abbüden. Weiter ist eine zweite Röntgenquelle 12' und ein zweiter Röntgendetektor 13' vorgesehen, die an einer Haltevorrichtung 11' angeordnet sind und Röntgenaufnahmen des Untersuchungsobjekts 3 aus einer festen Röntgenposition r₄ erstellen können.

Die Röntgendetektoren 13, 13' können jeweils ein Röntgenbildverstirlcer mit einer daran angeschlossenen Fernsebkette sein, deren Ausgangssignale von einem Analog-DigitalWandler 14 digitalisiert und in einem Speicher 15 gespeichert werden. Die von der bildgebenden Einheit 12,13 aus unterschiedlichen Röntgenpositionen r₁,... rᵢ,..... rₘ (von denen in der Zeichnung nur die Positionen r₁, r₂, r₃ explizit dargestellt sind) erfaßten Röntgenprojektionsbilder D₁,.... Dᵢ,... Dᵢₘ können von einer Bildverarbeitungseinheit 16 verarbeitet und einzeln oder als Bildfolge auf einem Monitor 18 dargestellt werden. Auch die von der bildgebenden Einheit 12', 13' aus der festen Rontgenposition r' zu diskreten Zeitpunkten t wäbrend eines Kontrastmittelbolus T aufgenommenen Röntgenprojektionsbilder E₀, E₁, E₂, ..., Eₙ können von der Bildverarbeitungseinheit 16 verarbeitet und auf dem Monitor 18 dargestellt werden. Die Steuerung der einzelnen Komponenten der Röntgeneinrichtung erfolgt mit Hilfe einer Steuereinheit 17.

Weiter ist eine Recheneinheit 19 vorgesehen, der die Röntgenprojektionsaufnahmen Dᵢ und E; zugeführt werden, welche daraus die zur Darstellung des zeitlichen Verlaufs des Blutflusses in den untersuchten Gefäßen des Untersuchungsobjekts 3 ermittelt. Diese können wiederum auf dem Monitor 18 dargestellt werden.

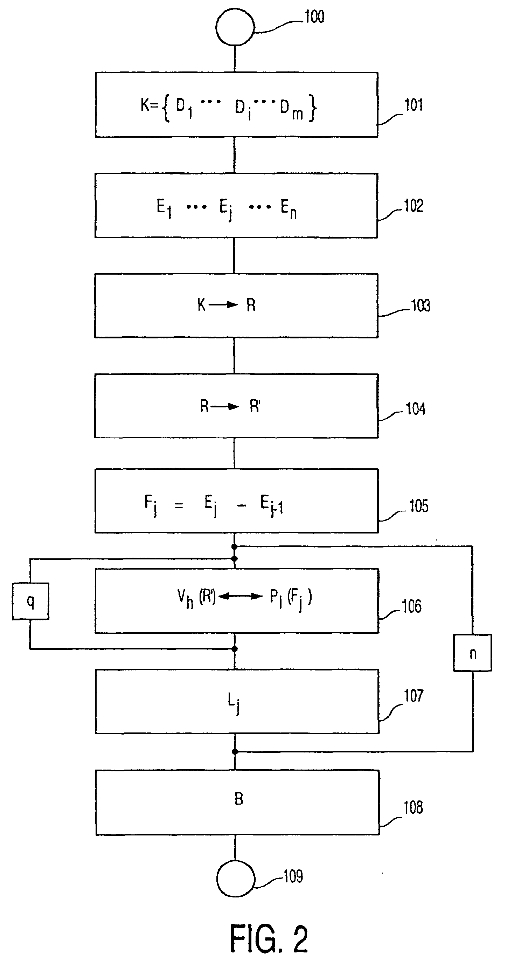

Anhand des in Fig 2 gezeigten Ablaufschemas einer ersten Ausgestaltung des erfindungsgemäßen Verfahrens soll die Erfindung näher erläutert werden. Nach der Initialisierung (Verfahrensschritt 100) wird nach einer Kontrastmittelinjektion der G Bogen 10 schrittweise um seinen Mittelpunkt bewegt und gleichzeitig eine Serie von m Röntgenprojektionsbildern Dᵢ erstellt (z.B. m= 100), die das Untersuchuagsobjekt 3 und die darin befindlichen mit einem Kontrastmittel gefüllten Blutgefäße aus unterschiedlichen Perspektiven darstellen (Schritt 101). Diese Röntgenprojektionsbilder Dᵢ bilden einen dreidimensionalen Röntgenbilddatensatz K.

In einem zeitgleich oder zeitlich später (nach einer erneuten Kontrastmittelinjektion) stattfindenden Verfahrensschritt 102 wird mittels der bildgebenden Einheit 12', 13' aus einer festen Perspektive eine zweite Serie von Röntgenprojektionsbildern Eⱼ aufgenommen- Im nachfolgenden Verfahrensschritt 103 werden einerseits Abbildungsfehler korrigiert, die beispielsweise durch die Unvollkommenheit des Bildaufnehmers oder durch die mechanische Verformung des C-Bogens zustande kommen. Andererseits wird aus den Röntgenprojektionsausnahmen Dᵢ ein dreidimensionales Konstruktionsbild R mittels eines bekannten Rekonstruktionsalgorithmus erstellt. Aus diesem Rekonstruktionsbild R wird im Verfahrensschritt 104 ein Teilrekonstruktionsbild R' ermittelt, das im wesentlichen oder ausschließlich Informationen über den Verlauf der Blutgefäße in dem untersuchten Bereich enthält. Dieses Teilrekonstruktionsbild R enthält q Voxel Vₖ, die durch ihre Koordinaten im Raum gekennzeichnet sind.

In einem weiteren Verfahrensschritt 105 werden aus jeweils zwei zeitlich aufeinanderfolgend aufgenommenen Röntgenprojektionsbildern Eⱼ₋₁ und Eⱼ Differenzbilder Fⱼ ermittelt, die nur noch die Information enthalten, welchen Weg das Kontrastmittel in dem Zeitraum zurückgelegt hat, der zwischen der Erstellung der beiden Röntgenprojektionsbildern Eⱼ₋₁ und Eⱼ liegt.

Nachfolgend wird jedes der n Differenzbilder Fⱼ mit dem Teilrekonstruktionsbild R' verglichen, wodurch dieses zeitlich kodiert wird, Dazu wird im Verfahrensschritt 106 jedes der q Voxel Vₖ des Teilrekonstruktionsbildes R' auf jedes Differenzbild Fⱼ projiziert d.h. es wird aus dem Teilrekonstruktionsbild R' ein Pseudo-Projektionsbild berechnet und mit den einzelnen Differenzbildern Fⱼ verglichen. Zur Berechnung dieses Pseudo-Projektionsbildes muß die Geometrie der bildgebenden Einheit 12', 13' bekannt sein, damit das Koordinatensystem des Pseudo-Projektionsbildes mit dem Koordinatensystem der Röntgenprojektionsbilder Eⱼ und der Differenzbilder Fⱼ übereinstimmt

Durch den Vergleich der Voxel Vₖ des Teilrekonstruktionsbildes R' bzw. der Pixel des Pseudo-Projektionsbildes mit den Pixeln P₁ der einzelnen Differenzbilder F; werden jeweils die Voxel des Teilrekonstruktionsbildes R' markiert, für welche das entsprechende Differenzbild Fⱼ ein korrespondierendes Pixel aufweist Diese markierten Voxel werden in Verfahrensschritt 107 zu einem Teilvoxelsatz Lⱼ zusammengefaßt

Vereinfacht gesagt wird in den Verfahrensschritten 106 und 107 überprüft in welchen Abschnitt des Gefäßsystems das Kontrastmittel in einem Zeitintervall zwischen der Erstellung zweier Röntgenprojektionsbilder Eⱼ₋₁ und Eⱼ vorgedrungen ist bzw. aus welchem Abschnitt des Gefäßsystems das Kontrastmittel in diesem Zeitintervall abgeflossen ist, und die Voxel in dem Teilrekonstruktionsbild R', die diesen Gefäßabschnitt darstellen, werden entsprechend markiert und als Teilvoxelsatz Lⱼ zusammengefaßt Dieser Verfahrensschritte wird n-mal durchgeführt, also für jedes Differenzbild Fⱼ einmal. Daraus ergeben sich n Teilvoxelsätze Lⱼ, aus denen sich im Verfahrensschritt 108 ein oder mehrere Bilder B erstellen lassen, die den zeitlichen Verlauf des Blutflusses darstellen. Beispielsweise können die Voxel jedes Teilvoxelsatzes Iⱼ farblich anders dargestellt in einem Gesamtbild gezeigt werden. In Verfahrensschritt 109 ist das Verfahren schließlich beendet.

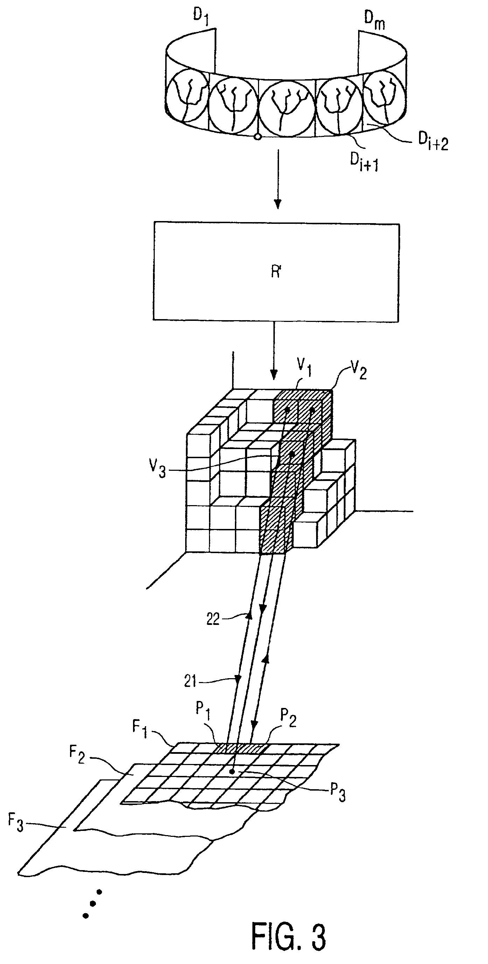

Anhand der Figur 3 soll insbesondere der Verfahrensschritt 106 nochmals veranschaulicht werden. Nachdem aus den Röntgenprojektionsbildern Dᵢ in mehreren Schritten ein Teilrekonstruktionsbild R' ermittelt wurde, das nur noch die Information über die Gefäßstruktur enthält, werden die Pixel Vₖ dieses Teilrekonstruktionsbild R' auf die Differenzbilder Fⱼ projiziert. Bei der Projektion auf das erste Differenzbild F₁ wird das Voxel V₁ entlang des Projektionsstrahls 21 auf das Pixel P₁ projiziert. Anhand der Graufärbung, die verdeutlichen soll, daß ein Voxel bzw. ein Pixel einen Teil einer Gefäßstruktur darstellt, was beispielsweise anhand des über oder unter einem bestimmten Grenzwert liegende Bildwertes festgestellt werden kann, ist erkennbar, daß zu dem Voxel V₁ ein korrespondierendes Pixel P₁ in dem Differenzbild F₁ existiert. Dies bedeutet, daß der durch das Voxel V₁ dargestellte Abschnitt des Gefäßsystems in dem zu dem Differenzbild F₁ korrespondierenden Zeitraum mit Kontrastmittel gefüllt worden ist. Das Voxel V₁ wird deshalb markiert, was symbolisch durch den Pfeil 22 gezeigt ist.

Entsprechendes gilt für das Voxel V₂, das auf das Pixel P₂ projiziert und ebenfalls markiert wird. Das Voxel V₃, das auf das Pixel P₃ projiziert wird, wird dagegen nicht markiert, da in dem Differenzbild F₁ das Pixel P₃ bzw. der durch dieses Pixel dargestellte Teil des Untersuchungsobjekts nicht mit Kontrastmittel gefüllt worden ist.

Nach dieser Methode werden alle Voxel Vₖ des Teilrekonstruktionsbildes R' oder zumindest alle Voxel, die das Gefäßsystem abbilden, nacheinander mit den Pixeln jedes einzelnen Differenzbildes Fⱼ verglichen und markiert. In dem gezeigten Beispiel bilden die Voxel V₁ und V₂, die nach dem Vergleich mit dem Differenzbild F₁ als einzige markiert sind, den Teilvoxelsatz L₁, der in einem am Ende des Verfahrens erstellten dreidimensionalen Rekonstruktionsbild mit einer ersten Farbe dargestellt werden kann. Der Teilvoxelsatz L₁ enthält also alle Voxel, in die in dem Zeitraum zwischen der Erstellung der Röntgenprojektionsbilder E₀ und E₁ Kontrastmittel eingeströrnt ist

Ein Teilrekonstrubaonsbild R' kann auch auf andere Weise als anhand von Fig. 2 beschrieben erhalten werden, beispielsweise durch Subtraktionsangiographie. Dazu werden mittels der bildgebenden Einheit 12, 13 zwei Serien von Röntgenprojektionsbildern aus unterschiedlichen Röntgenpositionen erstellt, einmal mit und einmal ohne Kontrastmittelzuführung. Die jeweils aus derselben Röntgenposition erstellten Röntgenprojektionsbilder der beiden Serien werden dann voneinander subtrahiert, und die resultierenden Projektionsbilder werden zu dem gewünschten Teilrekonstruktionsbild R' rekonstruiert.

Im Folgenden wird eine weitere Ausgestaltung des erfindungsgemäßen Verfahrens beschrieben. Diese unterscheidet sich von der in Fig. 2 gezeigten Ausgestaltung zunächst dadurch, daß nur eine einzige Serie von Röntgenprojektionsbildern Dᵢ ermittelt wird; es werden also keine Röntgenprojektionsbilder Eⱼ aus einer festen Röntgenposition ermittelt, so daß auch die zweite bildgebende Einheit 12', 13' (siehe Fig. 1) entfallen kann. Ansonsten entsprechen die Verfahrensschritte 200 bis 203 unverändert den Verfahrensschritten 100 bis 104. wobei Verfahrensschritt 102 entfällt.

Im anschließenden Verlährensschritt 204 werden die Röntgenprojektionsbilder Dᵢ segmentiert, d.h., es werden en den einzelnen Bilden Dᵢ die die Gefäßstrukturen darstellenden Pixel ausgewählt und auschließlich in den segmentierten Röntgenprojektionsbildern Dᵢ' dargestellt, während andere Bildelemente, wie z.B. der Hintergrund oder andere Organe, eliminiert werden. Dies kann beispielsweise mittels der Bildwerte erfolgen, die für die mit Kontrastmittel gefüllten Gefäße über bzw. unter einem bestimmten Grenzwert liegen, während dies für andere Bildelemente in der Regel nicht der Fall ist

Diese segmentierten Röntgenprojektionsbilder Dᵢ' werden nachfolgend einzeln nacheinander nouit dem Teilrekonstruktionsbild R' verglichen, bzw. es werden die Pixel P₁ der segmentierten Röntgenprojektionsbilder Dᵢ' mit den Voxeln Vₖ des Teilrekonstruktionsbildes R' verglichen. Dies erfolgt auf die gleiche Weise, wie anhand von Fig. 3 für die Differenzbilder Fⱼ beschrieben wurde. Ebenso erfolgen die Erstellung der Teilvoxelsätze Lⱼ und die Erstellung der Bilder B zur Darstellung des Verlaufs des Blutflusses in den Verfahrenschritten 206 und 207 auf die gleiche Weise wie bereits oben beschrieben. Im Verfahrensschritt 208 ist das Verfahren wiederum beendet.

Zwar hat diese Ausgestaltung gegenüber der anhand von Fig. 2 erläuterten Ausgestaltung den Vorteil, daß nur eine bildgebende Einheit (12, 13) erforderlich ist und daß nur eine einzige Serie von Röntgenprojektionsbildern Dᵢ erstellt werden muß. Die Segmentierung dieser Bilder Dᵢ in Verfahrensschritt 204 kann jedoch in der Praxis schwierig sein und zu Ungenauigkeiten führen. Außerdem ist der Informationsgehalt in den segmentierten Röntgenprojektionsbildern Dᵢ' ein anderer als in den Differenzbildern Fⱼ, die die zeitliche Veränderung des Blutflusses in einem Zeitintervall enthalten, während die segmentierten Röntgenprojektionsbilder Dᵢ' den gesamten mit Kontrastmittel gefüllten Bereich des Gefäßsystems zeigen.

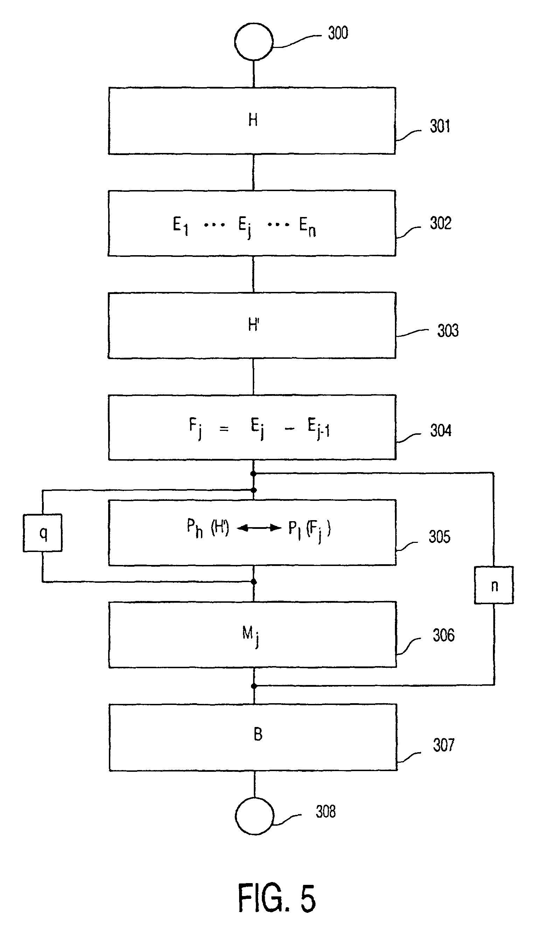

Eine dritte Ausgestaltung des erfindungsgemäßen Verfahrens soll anhand von Fig. 5 erläutert werden. Nach dem Verfährensstart in Schritt 300 wird ein zweidimensionaler Röntgenbilddatensatz H in Verfahrensschritt 301 ermittelt. Dieser kann ein einziges den komplett mit Kontrastmittel gefüllten Gefäßbaum darstellendes Röntgenprojektionsbild oder ein aus mehreren einzelnen Röntgenprojektionsbildern zusammengesetztes Bild sein. Dieser Datensatz H kann auch zu einem früheren Zeitpunk bereits ermittelt worden sein. In Verfahrensschritt 302 wird mittels der bildgebenden Einheit (12,13) eine Serie von Röntgenprojektionsbildern Eⱼ aus einer festen Röntgenposition nach einer Kontrastinjektion erstellt. Zeitgleich oder auschließend wird in Verfahrensschritt 303 der zweidimensionale Bilddatensatz H segmentiert, so daß in dem segmentierten Bili:1c1musarz H' nur noch die Gefäßstrukturen enthalten sind.

Nach n-maliger Durchführung der Verfahrensschritte 305 und 306 ist das gesamte segmentierte Bild H' zeitlich kodiert, und es können ein oder mehrere Bilder zur Veranschaulichung des zeitlichen Verlaufs des Blutflusses in diesem Bild dargestellt werden (Schritt 307), wonach das Verfahren beendet ist (Schritt 308).

Eine Ausgestaltung der Erfindung ist besonders für die zweidimensionale Rotationsangiographie geeignet. Diese liefert bereits einen zweidimensionalen Röntgenbildatensatz, beispielsweise ein zweidimensionales Röntgenprojektionsbild, das den mit Kontrastmittel gefüllten Gefäßbaum komplett enthält und ein bereits früher erstellter oder ein aktueller Röntgenbilddatensatz sein kann. Die aktuellen während einer Kontrastmittelzuführung aus einer festen Röntgenposition ermittelten Röntgenprojektionsbilder werden gemäß dieser Ausgestaltung voneinander subtrahiert, so daß jedes Differenzbild die Information enthält, welchen Weg das Kontrastmittel in dem Gefäßbaum zwischen den zwei Zeitpunkten zurückgelegt hat, zu denen die beiden voneinander subtrahierten Röntgenprojektionsbilder ermittelt wurden. Diese Differenzbilder werden dann zur zeitlichen Kodierung des Röntgenbilddatensatzes benutzt

Bei einer bevorzugten Weiterbildung werden die Röntgenprojektiansbilder aus unterschiedlichen Positionen ermittelt. Der Bilddatensatz ist dabei ein aus diesen Rötitgenprojektionsbildem ermittelter dreidimensionaler Räntgenbilddatensatz. Bei dieser Weiterbildung wird somit nur eine einzige Serie von Röntgenprojektionsbildern aus unterschiedlichen Richtungen ermittelt, die auch die zeitliche Information enthalten. Nach Segmentierung der Blutgefäße in diesen Röntgenprojektionsbildern werden diese mit dem Röntgenbilddatensatz beispielsweise dadurch verglichen und zeitlich kodiert, daß mit Hilfe der bekannten Abbildungsgeometrie der Röntgeneinrichtung Pseudo-Projektionsbilder aus dem Röntgenbilddatensatz berechnet und mit den tatsächlichen Röntgenprojektionsbildern verglichen werden.

Eine besonders vorteilhafte Weiterbildung, die ebenfalls für die dreidimensionale Rotationsangiographie geeignet ist, ist wie folgt angegeben. Dabei werden zwei Serien von Röntgenprojektionsbildern ermittelt, die erste Serie aus einer festen Röntgenposition, die zweite Serie aus unterschiedlichen Röntgenpositionen. Dies kann entweder zeitlich nacheinander erfolgen, wobei allerdings zwei Kontrastmittelinjektionen erforderlich sind, oder auch zeitgleich mit einer Röntgeneinrichtung mit zwei bildgebenden Einheiten. Aus den Röntgenprojektionsbildern der ersten Serie werden Differenzbilder ermittelt, die wie oben beschrieben die zeitliche Information tragen, während aus den Rontgenprojekdonsbildem der ersten Serie mittels eines bekannten Rekonstruktionsalgorithmus ein dreidimensionaler Röntgenbilddatensatz ermittelt wird. Dieser wird dann mittels der Diffaenzbilder zeitlich kodiert. Diese Weiterbildung, insbesondere die Bildung von Differenzbildern als Träger der zeitlichen Information, hat den Vorteil, daß in den Differenzbildern immer nur die zeitliche Veränderung des Kontrastmittelflusses (= des Blutflusses) in einem bestimmten Zeitintervall enthalten ist, während bei unmittelbarer Ausnutzung der Röntgenprojektionsbilder als Träger der zeitlichen Information immer die gesamte Verteilung des Kontrastmittels in dem Gefäßsystem enthalten und deshalb die Information wesentlich ungenauer ist. Dies hängt auch damit zusammen, daß sich das Kontrastmittel sehr schnell in dem gesamten zu betrachtenden Gefäßsystem verteilt, so daß die Unterschiede der Verteilung des Kontrastmittels zwischen zwei relativ kurz aufeinanderfolgenden Zeitpunkten nur relativ gering sind.

Im Folgenden sind bevorzugte Möglichkeiten angegeben, nach denen die zeitliche Kodierung des Bilddatensatzes und der Vergleich des Bilddatensatzes mit den segmentierten Röntgenprojektionsbildern erfolgen kann. Die angegebenen Verfahrensschritte entsprechen dabei dem aus der EP 880 109 A2 bekannten Verfahren, auf deren Offenbarungsgehalt hiermit ausdrücklich Bezug genommen wird und der als in die vorliegende Anmeldung mit aufgenommen gelten soll Aus dieser Druckschrift ist ein Verfahren zur Ermittlung der räumlichen Transformation zwischen einem durch einen Datensatz dreidimensional abgebildeten Objekt und dem Objekt selbst bekannt Von einem Teil des durch den Datensatz darstellten Volumens wird dort ein Pseudo-Projektionsbild berechnet und mit einer Röntgenprojektionsaufnahme des Objekts selbst verglichen. Die Parameter, die der Berechnung des Pseudo-Projektionsbildes zugrunde liegen, werden dabei so lange variiert, bis sich eine optimale Übereinstimmung ergibt. Dieses Verfahren kann bei der vorliegenden Erfindung vorteilhaft und in entsprechender Weise angewendet werden.

Vorteilhafte Weiterbildungen, betreffend die Darstellung des zeitlichen Verlaufs des Blutflusses, sind wie folgt angegeben. Beispielsweise kann die Zeitinformation in eine Farbkodierung umgewandelt werden, so daß der komplette zwei- oder dreidimensionale Datensatz mit der entsprechenden Farbkodierung als ein Bild dargestellt werden kann.

Die kodierten Teilpixelsätze bzw. Teilvoxelsätze können auch zeitlich nacheinander z.B. in einer Endlosschleife dargestellt werden, so daß der Eindruck Vermittelt wird, als ob das Blut durch die Blutgefäße fließen würde. Denkbar ist auch, Darstellungen aus beliebigen Betrachtungswinkeln zu ermöglichen oder die Darstellung rotieren zu lassen.

Die gezeigte Röntgeneinrichtung und die gezeigten Ausgestaltungen des erdindungsgemäßen Verfahrens sind lediglich beispielhaft zu verstehen. Die Röntgeneinrichtung kann auch anders ausgestaltet sein. Auch einzelne Verfahrensschritte, wie beispielsweise die Ermittlung von Röntgenprojektionsbildern, kann in der Praxis anders erfolgen.

## Patentansprüche

1. Röntgeneinrichtung
• mit einer Bildgebungseinheit (1) umfassend Röntgenprojektionsbilder (Dᵢ; Eᵢ) und einen Bilddatensatz (H; K), mit einer Röntgenquelle (12) und einem Röntgendetektor (13) zur Ermittlung einer Serie von Röntgenprojektionsbildern (Dᵢ; Eᵢ) während einer Kontrastmittelzuführung an die Blutgefäße und zur Ermittlung eines den Verlauf von Blutgefäßen in dem Untersuchungsobjekt (3) enthaltenden Bilddatensatzes (H; K),
• mit einer Recheneinheit (19) zur Segmentierung der mit Kontrastmittel gefüllten Bereiche der Blutgefäße in den einzelnen Röntgenprojektionsbildern (Dᵢ; Eⱼ) und zur zeitlichen Kodierung des Bilddatensatzes (H; K) durch Vergleich des Bilddatensatzes (H; K) mit segmentierten Röntgenprojektionsbildern (Dⱼ'; Fⱼ), dazu ausgebildet, die Röntgenprojektionsbilder (Eⱼ) aus einer festen Röntgenposition (r₄) zu ermitteln, wobei der Bilddateasatz ein zweidimensionaler Röntgeabilddatensatz (H) ist, dazu ausgebildet, dass zur Segmentierung der Röntgenprojektionsbdder (Eⱼ) Differenzbilder (Fⱼ) aus jeweils zwei zeitlich aufeinander folgenden Röntgenprojektionsbildern (Eⱼ) ermittelt werden und dass die zeitliche Kodierung durch Vergleich des zweidimensionalen Röntgenbilddatensatzes (H) mit den Differenzbildern (Fⱼ) erfolgt,
• mit einer Wiedergabeanordnung zur Darstellung eines oder mehrerer den zeitlichen Verlauf des Blutflusses darstellender Projektionsbilder (B),
**dadurch gekennzeichnet,**
• **dass** die Recheneinheit (19) der Röntgeneinrichtung derart ausgebildet ist, dass ein die Information über den Verlauf der Blutgefäße enthaltender Teil-Röntgenbilddatensatz (H') aus dem Röntgenbilddatensatz (H) segmentiert wird, dass die Pixel (Pₖ) des Teil-Röntgenbilddatensatzes (H') auf Differenzbilder (Fⱼ) aus jeweils zwei zeitlich aufeinander folgenden Röntgenprojektionsbildern (Eⱼ) projiziert werden, dass für die einzelnen Differenzbilder (Fⱼ) zugeordnete Teilpixelsätze (Mⱼ) aus den Differenzbildern F(j) ermittelt werden, wobei einem Teilpixelsatz (Mⱼ), welcher die Information enthält, welche Pixel des segmentierten Teil-Röntgenbilddatensatzes (M') einen Teil des Grefäßsystems zeigen, das sich in einem bestimmten Zeitintervall mit Kontrastmittel gefüllt hat bzw. aus dem in diesem Zeitintervall Kontrastmittel abgeflossen ist, diejenigen Pixel (Pₖ) des Teil-Röntgenbilddatasatzes (H') zugeordnet werden, für welche das zugehörige Diffesenzbild (Fⱼ) korrespondierende Pixel (P₁) aufweist, und dass aus den Teilpixelsätzen (Mⱼ) ein oder mehrere zweidimensionale Projektionsbilder (B) erstellt werden.

2. Röntgeneinrichtung nach Anspruch 1,
**dadurch gekennzeichnet,**
**dass** die Bildgebungseinheit eine erste Röntgenquelle (12) und einen ersten Röntgendetektor (13) zur Ermittlung einer ersten Serie von zweidimensionalen Röntgenbildern (Eⱼ) aus einer festen Röntgenposition (r₄) und eine zweite Röntgenquelle (12') und einen zweiten Röntgendetektor (13') zur Ermittlung einer zweiten Serie von zweidimensionalen Rantgenbüdern (Dᵢ) aus unterschiedlichen Röntgenpositionen (r₁, r₂, r₃) aufweist.

## Claims

1. An X-ray device
• comprising an image generating unit (1) comprising X-ray projection images (Dᵢ; Eⱼ) and an image data set (H; K), with an X-ray source (12) and an X-ray detector (13) for detecting a series of X-ray projection images (Dⱼ; Eⱼ) while a contrast medium is being administered to the blood vessels and for determining an image data set (H; K) containing the course of blood vessels in the object to be examined (3),
• comprising a calculation unit (19) for segmenting regions of the blood vessels filled with the contrast medium into the individual X-ray projection images (Dⱼ; Eⱼ) and for coding over time the image data set (H; K) by comparison of the image data set (H; K) with segmented X-ray projection images (Dᵢ; Fⱼ), arranged for determining the X-ray projection images (Eⱼ) from a fixed X-ray position (r4) where the image data set is a two-dimensional X-ray image data set (H), configured in such a way that for segmenting the X-ray projection images (Eⱼ) difference images (Fⱼ) are determined each time from two time-consecutive X-ray projection images (Eⱼ) and that the time coding is effected by comparison of the two-dimensional X-ray image data set (H) with the difference images (Fⱼ),
• comprising a display arrangement for displaying one or various projection images (B) displaying the time-dependent course of the blood flow,
**characterized in that**
• the arithmetic unit (19) of the X-ray device is arranged such that an X-ray image data sub-set (H') containing the information about the course of the blood vessels is segmented from the X-ray image data set (H), **in that** the pixels (Pₖ) of the X-ray image data sub-set (H') are projected on difference images (Fⱼ) from each time two time-consecutive X-ray projection images (Eⱼ), **in that** for the individual difference images (Fⱼ) associated pixel sub-sets (Mⱼ) are determined from the difference images (Fⱼ), where one pixel sub-set (Mⱼ), which contains the information about what pixels of the segmented X-ray image data sub-set (H') show a part of the vascular system that has filled with contrast medium in a certain time interval, or from which contrast medium has drained in this time interval, is assigned those pixels (Pₖ) of the X-ray image data sub-set (H') for which the associated difference image (Fⱼ) has corresponding pixels (P_{I}) and **in that** one or more two-dimensional projection images (B) are established from the pixel sub-sets (Mⱼ).

2. An X-ray device as claimed in claim 1, **characterized in that** the imaging unit has a first X-ray source (12) and a first X-ray detector (13) for determining a first series of two-dimensional X-ray images (Eᵢ) from a first fixed X-ray position (r₄) and a second X-ray source (12') and a second X-ray detector (13') for determining a second series of two-dimensional X-ray images (Dᵢ) from different X-ray positions (r₁, r₂, r₃).

## Revendications

1. Dispositif radiographique
• avec une unité d'imagerie (I) comprenant des images de projection radiographique (Dᵢ, Eᵢ) et un jeu de données d'image (H; K) avec une source de rayons X (12) et un détecteur de rayons X (13) pour la détermination d'une série d'images de projection radiographique (Dᵢ, Eᵢ) pendant l'apport de produit de contraste aux vaisseaux sanguins et en vue de la détermination d'un jeu de données d'images (H; K) contenant le tracé de vaisseaux sanguins dans l'objet d'examen (3),
• avec une unité de calcul (19) pour la segmentation des zones remplies de produit de contraste des vaisseaux sanguins dans les différentes images de projection radiographique (Dᵢ; Eⱼ) et pour le codage temporel du jeu de données d'image (H; K) par comparaison du jeu de données d'image (H; K) avec des images segmentées de projection radiographique (Dⱼ', Fⱼ), conçue pour déterminer les images de projection radiographique (Eⱼ) à partir d'une position radiographique fixe (r₄),
le jeu de données d'image étant un jeu de données d'image radiographique bidimensionnel (H) conçu pour que des images différentielles (Fⱼ) soient déterminées en vue de la segmentation des images de projection radiographique (Eⱼ) à partir de deux images de projection radiographique successives dans le temps et que le codage temporel intervienne par comparaison du jeu de données d'image radiographique bidimensionnel (H) avec les images différentielles (Fⱼ);
• avec un dispositif de reproduction pour la représentation d'une ou plusieurs images de projection (B) représentant le tracé temporel du flux sanguin,
**caractérisé en ce**
• **que** l'unité de calcul (19) du dispositif radiographique est conçue de telle sorte qu'un jeu de données d'image radiographique partielles (H') contenant des informations à propos du tracé des vaisseaux sanguins soit segmenté d'un jeu de données d'image radiographique (H), que les pixels (Pₖ) du jeu de données d'image radiographique partielles (H') sont projetés sur des images différentielles (Fⱼ) à partir de deux images de projection radiographique (Eⱼ) successives dans le temps, que les jeux de pixel partiels (Mⱼ) affectés aux images différentielles (Fⱼ) individuelles sont déterminés à partir des images différentielles (Fⱼ), auquel cas on affecte à un jeu de pixel partiel (Mⱼ) qui contient l'information des pixels du jeu de données d'image radiographique partiel (M') segmenté qui montre une partie du système vasculaire qui s'est rempli de produit de contraste ou s'est vidé du produit de contraste durant cet intervalle temporel les pixels (Pₖ) du jeu de données d'image radiographique partielles (H') pour lequel l'image différentielle (Fⱼ) correspondante présente des pixels correspondants (Pₗ) et qu'une ou plusieurs images de projection (B) bidimensionnelles sont établies à partir des jeux de pixel partiels (Mᵢ).

2. Dispositif radiographique selon la revendication 1,
**caractérisé en ce**
• **que** l'unité d'imagerie présente une première source de rayons X (12) et un premier détecteur de rayons X (13) pour la détermination d'une première série d'images radiographiques (Eⱼ) bidimensionnelles à partir d'une position radiographique fixe (r₄) et d'une deuxième source radiographique (12') et un deuxième détecteur de rayons X (13') pour la détermination d'une deuxième série d'images radiographiques (Dᵢ) bidimensionnelles à partir de différentes positions radiographiques (r₁, r₂, r₃).
